Europäisches Patentamt

**(19)** European Patent Office

Office européen des brevets

**(11)** Publication number : **0 636 365 A1**

**(12)** # EUROPEAN PATENT APPLICATION

**(21)** Application number : **94305535.0**

**(22)** Date of filing : **27.07.94**

**(51)** Int. Cl.[6] : **A61K 9/20, A61K 9/50**

**(30)** Priority : **27.07.93 US 98019**

**(43)** Date of publication of application :
**01.02.95 Bulletin 95/05**

**(84)** Designated Contracting States :
**AT BE CH DE DK ES FR GB GR IE IT LI LU MC NL PT SE**

**(71)** Applicant : **McNEIL-PPC, INC.**
**Van Liew Avenue**
**Milltown New Jersey 08850 (US)**

**(72)** Inventor : **Gole, Dilip J.**
**3050 Bolgos Circle**
**Ann Arbor, MI 48105 (US)**
Inventor : **Reo, Joseph**
**342 Courtland Ave.**
**Harleysville, PA 19483 (US)**
Inventor : **Roche, Edward J.**
**1849 Hawthorne Place**
**Paoli, PA 19301 (US)**
Inventor : **Wilkinson, Paul K.**
**5341 Salsburg Court**
**Ann Arbor, MI 48103 (US)**

**(74)** Representative : **Mercer, Christopher Paul et al**
**Carpmaels & Ransford**
**43, Bloomsbury Square**
**London WC1A 2RA (GB)**

**(54) Freeze-dried pharmaceutical dosage form and process for preparation thereof.**

**(57)** The present invention relates to a freeze-dried pharmaceutical dosage form containing a porous matrix of a water-soluble or water-dispersible carrier material containing a coated pharmaceutical particle. The pharmaceutical granule is coated with a blend of a first polymer selected from the group consisting of cellulose acetate and cellulose acetate butyrate and a second polymer selected from the group consisting of polyvinyl pyrrolidone and hydroxypropyl cellulose.

EP 0 636 365 A1

## FIELD OF THE INVENTION

The present invention relates to a freeze-dried pharmaceutical dosage form containing coated pharmaceutical particles and to a process for preparing such dosage forms.

## BACKGROUND OF THE INVENTION

Freeze-dried or lyophilized pharmaceutical dosage forms generally consist of a porous matrix of a water-soluble or water-dispersible carrier material which is impregnated with a unit dose of the pharmaceutical active. These dosage forms are prepared by first adding the pharmaceutical active to a solution comprising the carrier material and a suitable solvent, typically water. The resulting composition is then subjected to a freeze drying procedure whereby the solvent sublimes under a high vacuum.

The porous matrix of the carrier material obtained through the freeze-drying process resembles a solid foam having interstices dispersed throughout. When the dosage form is subjected to a liquid, the liquid permeates the product and causes a carrier material to rapidly disintegrate or dissolve. This results in rapid release of the pharmaceutical active or other material embodied within the matrix. These products are considered to be fast dissolving dosage forms because they generally disintegrate in water or on the tongue within 10 seconds, and are particularly suitable for patients who have difficulty swallowing conventional tablets or capsules.

It is generally preferred for freeze-dried dosage forms that the pharmaceutical be water insoluble and be relatively tasteless. Problems can arise for soluble drugs due to formation of eutectic mixtures, which lower the freezing point of the formulation, resulting in an incomplete freezing or melting during the freeze-drying process. The phenomenon results in product loss. Since the freeze-dried dosage form disintegrates rapidly in the mouth, rather than being swallowed as in the case of tablets or capsules, the pharmaceutical active should be substantially taste-free in order to obtain a product which is palatable to the consumer.

Attempts have been made in freeze-dried dosage forms to mask the bitter or otherwise disagreeable taste of the pharmaceutical by shielding the pharmaceutical with a coating. Blank, et al., in U.S. Patent Nos. 4,760,093; 4,760,094; and 4,767,789 suggest the use of a taste-neutral powder form of acetaminophen in freeze-dried dosage forms, which is obtained by spray-drying a suspension of acetaminophen in a solution of ethylcellulose or a copolymer based on dimethylaminoethyl methacrylate and neutral methacrylic acid esters. In each of the aforementioned patents, however, when the spray-dried acetaminophen was employed in a freeze-dried dosage form, the resulting product was found to have a bitter aftertaste.

Other coating systems have been suggested to taste-mask pharmaceuticals used in freeze-dried dosage forms. U.S. Patent Nos. 4,771,077; 4,835,186; and 4,835,187 to Reuter, et al., discuss the use of certain cellulose materials as coatings for taste-masking pharmaceuticals. In particular, it is suggested in these patents that acetaminophen can be rendered taste-neutral by spray-drying a suspension of colloidal silica in a lower alkanol solution of acetaminophen and ethyl cellose. Ibuprofen is said to be rendered taste-neutral by spray-drying a suspension of colloidal silica in a lower alkanol solution of ibuprofen and cellulose acetate phthalate. Taste-neutral spray dried powders of ibuprofen may also be obtained by spray-drying a suspension of colloidal silica in a lower alkanol solution of ibuprofen and ethyl cellulose, hydroxyethyl cellulose, hydroxypropylmethyl cellulose and admixtures thereof. These patents report, however, that when these spray-dried acetaminophen and ibuprofen powders are used in a freeze-dried dosage form, the resulting product had a bitter aftertaste.

Ho, et al., in U.S. Patent No. 4,035,188 discusses spray-drying a dispersion of ibuprofen and ethyl cellulose having a plasticizer dissolve or suspended therein for the purposes of taste-masking. When the resulting spray-dried product was used in a freeze-dried dosage form, the wafer was reported to have a bitter aftertaste.

A need, therefore, exists for a freeze-dried dosage form containing a pharmaceutical which has been coated with a material that effectively masks the bitter or otherwise objectionable taste of the pharmaceutical. Further, if the pharmaceutical is soluble or partially soluble in the solution of the carrier material, it would be advantageous that the coating be sufficiently insoluble so as to prevent leaching of the pharmaceutical, into the carrier solution to prevent the formation of eutectic mixtures.

## SUMMARY OF THE INVENTION

The present invention provides a freeze-dried dosage form containing a pharmaceutical coated with a material that provides taste-masking and protection against the leaching of the pharmaceutical into the solution of the carrier material during the freeze-drying process. In a preferred embodiment of the present invention, the freeze-dried pharmaceutical dosage form contains a porous matrix of a water-soluble or water-dispersible carrier material which contains at least one coated particle. The coated particle contains at least one pharmaceutical coated with a blend of a first polymer selected from the group consisting of cellulose acetate and cel-

EP 0 636 365 A1

lulose acetate butyrate and a second polymer selected from the group consisting of polyvinyl pyrrolidone and hydroxypropyl cellulose, where the weight ratio of the first polymer to the second polymer is within the range of about 90:10 to about 50:50.

In a further preferred embodiment of the present invention, the freeze-dried pharmaceutical dosage form is prepared by coating the pharmaceutical with the aforementioned blend of first and second polymers in a fluidized bed coating operation, thereby forming the coated pharmaceutical particles. A suspension of the coated particles is then formed with a solution comprising a water-soluble or water-dispersible carrier material and a solvent. A unit volume of the suspension is freeze-dried to form a porous matrix of the water-soluble or water-dispersible carrier material with the coated particles contained therein.

## DETAILED DESCRIPTION OF THE PREFERRED EMBODIMENTS

The pharmaceutical dosage forms of the present invention rapidly disintegrate or dissolve when contacted by water, saliva and aqueous solutions, and are therefore particularly useful in the oral delivery of drugs. These dosage forms generally disintegrate in the mouth within about 30 seconds, and preferably within about 10 seconds or less.

The dosage forms contain a porous matrix of a water-soluble or water-dispersible carrier material. This matrix contains interstices dispersed throughout and resembles a dried foam. When the porous matrix comes in contact with a liquid, the liquid enters the surface pores and moves rapidly through the interstices to the interior of the product. Permeation of the liquid through both the interior and exterior regions of the product results in rapid disintegration or dissolution of the carrier material. This disintegration or dissolution results in rapid release of a pharmaceutical contained within the matrix.

The carrier material used in the dosage form may be any pharmaceutically acceptable water-soluble or water-dispersible material which is capable, upon freeze-drying or lyophilization, to form a porous matrix that rapidly disintegrates or dissolves when contacted with a liquid. Materials suitable for use as the carrier include animal or vegetable proteins, such as gelatins, dextrins and soy; wheat and psyllium seed proteins; gums, such acacia, guar, agar and xanthan; polysaccharides; alginates; carboxymethyl celluloses; carrageenans; dextrans; pectins; synthetic polymers such as polyvinyl pyrrolidone; and poly-peptide/protein or polysaccharide complexes such as gelatin-acacia complexes. Other suitable carrier materials for forming the porous matrix include sugars, such as mannitol, dextrose, lactose and galactose; cyclic sugars, such as cyclodextrins, inorganic salts, such as sodium phosphate, sodium chloride and aluminum silicates; and amino acids having from 2 to about 12 carbon atoms, such as glycine and 1-alanine.

Preferred carrier materials include pharmaceutical grade gelatins and pectins (non-hydrolyzed, partially hydrolyzed or hydrolyzed), glycine, mannitol and mixtures thereof. A further preferred carrier material is a mixture of gelatin and one or more amino acids having from 2 to 12 carbon atoms, such as glycine.

The porous matrix in the freeze-dried pharmaceutical dosage form of the present invention is used to carry a coated particle containing at least one pharmaceutical active. As used in the present invention, "coated particle" refers to a solid pharmaceutical in the form of a crystal or particle, an agglomerate of individual particles, or a granulated particle, which has been coated. The pharmaceutical active is coated with a blend of a first polymer selected from the group consisting of cellulose base acetate and cellulose acetate butyrate and a second polymer selected from the group consisting of polyvinyl pyrrolidone and hydroxypropyl cellulose. The weight ratio of the first polymer to the second polymer in this blend is within the range of about 90:10 to about 50:50 and preferably about 90:10 to about 70:30.

The first polymer of the blend is generally water-insoluble, but is soluble in organic solvents. These polymers provide good taste-masking properties since they do not dissolve in the mouth. However, if used alone, they do not provide adequate bioavailability of the pharmaceutical. To provide the requisite bioavailability, a second polymer, which is soluble in both water and organic solvents, is added to the blend that is used to coat the pharmaceutical active. This blend of first and second polymers provides the balance needed for the taste masking, prevents leaching of the pharmaceutical when it is dispersed in the solution of the carrier material during the freeze-drying process and provides the necessary bioavailability of the pharmaceutical.

Preferred blends of the first and second polymers include cellulose acetate (CA) and polyvinyl pyrrolidone (PVP) having a weight ratio of CA:PVP within the range of about 90:10 to about 60:40, cellulose acetate (CA) and hydroxypropyl cellulose (HPC) having a weight ratio of CA:HPC within the range of about 90:10 to about 50:50, cellulose acetate butyrate (CAB) and hydroxpropyl cellulose (HPC) having a weight ratio of CAB:HPC within the range of about 90:10 to about 50:50, and cellulose acetate butyrate (CAB) and polyvinyl pyrrolidone (PVP) having a weight ratio of CAB:PVP within the range of about 90:10 to about 60:40.

Cellulose acetate NF powder, e.g., CA 398-10, CA 320-S or CA 435-75S available from FMC Corp., may be used as the first polymer in the blend. CA 398-10 polymer has an acetyl content of about 39.8 weight percent,

3

a hydroxyl content of about 3.4 weight percent, a degree of substitution of 2.7 and a solution viscosity of about 38 poises or 10 seconds, as determined by ASTM Method D 1343 in the solution described as Formula A, ASTM Method D 871. The typical weight average molecular weight, according to the manufacturer, is 177,000 and the typical number average molecular weight is 58,500. CA 320-S polymer has an acetyl content of about 32.0 weight percent, a hydroxyl content of about 9.0 weight percent and a degree of substitution of 2.1. In a solution of 90:10 $CH_2Cl_2$:methanol, at 4%(w/w) concentration, the viscosity is 50 centipoise. The typical weight average molecular weight is 100,500 and the typical number average molecular weight is 63,500. CA 435-75S has an acetyl content of about 43.6 weight percent and a hydroxyl content of about 0.9 weight percent.

Cellulose acetate butyrate, e.g., CAB 171-15S, CAB 381-2 and CAB 500-1 available from FMC Corp., may also be used as the first polymer. CAB 171-15S has a butyryl content of 17 weight percent, an acetyl content of 29.5 weight percent, a hydroxyl content of 1.5 weight percent and a viscosity of 24 centipoises in a 4 weight percent solution of methylene chloride:methanol (90:10) one day after solution preparation at 25°C. CAB 381-2 has a butyryl content of 37 weight percent, an acetyl content of 13 weight percent and a hydroxyl content of 1.5 weight percent. CAB 500-1 has a butyryl content of 50 weight percent, an acetyl content of 5 weight percent and a hydroxyl content of 0.5 weight percent.

Polyvinyl pyrrolidone (Povidone USP), e.g., PLASDONE® K-25, K-25/28 or K-29/32 from ISP Corporation, may be used as the second polymer in the blend. Povidone K-25 has a viscosity of 2.4 centipoises in a 5% solution of water at a pH 7 and 25°C.

Hydroxypropyl cellulose, e.g., KLUCEL EF, JF and LF, available from Aqualon Co. may also be used as the second polymer. These polymers generally have a molecular weight of about 80,000 to about 370,000.

The blend of first and second polymers may be coated directly onto the pure pharmaceutical (crystal, particle or agglomerated particles) or may be coated onto granulated particle containing the pharmaceutical. In the case of a granulated particle, such as a rotogranulated particle, the pharmaceutical active will constitute from about 5 to about 90 weight percent of the particle, with the remainder being the binder or filler. Suitable binders include polyvinyl pyrrolidone, hydroxypropylmethyl cellulose, hydroxypropyl cellulose, and other pharmaceutically acceptable polymers. Fillers suitable for use in such granulated particles include lactose, confectioner's sugar, mannitol, dextrose, fructose, other pharmaceutically acceptable saccharides and microcrystalline cellulose.

The coated particles are prepared by spraying an organic solvent solution of the polymeric blend onto the pharmaceutical, or a rotogranulated particle containing the pharmaceutical, in a fluidized bed, such as a Wurster coater or a rotogranulator. A wide variety of organic solvents may be used to prepare the solution of the polymeric blend. For example, a preferred solvent is a mixture of acetone and methanol, but other solvent systems may be employed, including methylene chloride, methylene chloride-methanol, acetone-ethyl acetate, toluene-ethanol and acetone-ethanol. Generally, the proportion of the polymer blend in the solvent solution will be within the range of about 5 to about 20, preferably about 8 to about 15, weight percent, depending on the solvent and other similar considerations.

When a fluidized bed coating operation is used, air, which may be heated, passes through a bed of the pharmaceutical solids to fluidize them, and the solution of the polymeric blend is sprayed onto the fluidized bed and thereby coats the pharmaceutical. The air passing through the bed dried the coating onto the pharmaceutical, so that a dry coated particle is obtained.

Conventional fluidized bed coating equipment is used in the present invention to coat the pharmaceutical or the granulated particle containing the pharmaceutical. This equipment includes Wurster fluid-bed coaters, where the solution of the polymer blend is sprayed from the bottom of the chamber, and a rotogranulator, where the solution of the polymer blend is tangentially sprayed. These coating operations are further described in Liberman et al., Pharmaceutical Dosage Forms, Marrel Dekker, Inc., New York, Vol. 3, pp. 138-150 (1990), which is hereby incorporated by reference.

Spray-dried coated particles are generally not used in the present invention because the resulting coating is not contiguous over the surface of the solid pharmaceutical. If a contiguous coating is not obtained, the solvent(s) used in the freeze-drying step can enter the coated particle and dissolve the pharmaceutical active. If such dissolution occurs, the finished dosage form will exhibit the undesirable, characteristic taste of the active ingredient. Further, the dissolved pharmaceutical may also affect the freezing and freeze-drying of the dosage form, resulting in a dosage form with undesirable characteristics.

Substantially all of the pharmaceutical or rotogranulated pharmaceutical should be coated with a layer of the blend of first and second polymers having a thickness of about 3 to about 10 microns. The coating should be substantially free of cracks, holes or other imperfections when examined under a scanning electron microscope at 100-500x. It is necessary to avoid such defects in the coating to prevent leaching of the pharmaceutical from the coated particle when the particle is suspended in the solution of the carrier material during the freeze-drying step.

4

The coated particle, in a dried state, generally contains about 5 to about 60, preferably about 15 to 40, weight percent of the blend of the first and second polymers. The exact proportions of the coating to the pharmaceutical can, however, vary depending upon the level of taste-masking required and whether a sustained or immediate release of the pharmaceutical is desired. Larger proportions of the coating tend to provide a sustained release effect and enhance taste-masking.

The freeze-dried dosage form of the present invention may be used to administer a wide variety of pharmaceutical actives. This dosage form is particularly useful for the oral administration of solid pharmaceuticals. Pharmaceutical actives which can be used in the dosage form include acetaminophen, ibuprofen, flurbiprofen, naproxen, aspirin, pseudoephedrine, chlorpheniramine maleate, dextromethorphan, diphenhydramine, famotidine, loperamide, ranitidine, cimetidine, astemizole, terfenadine, terfenadine carboxylate, domperidone, phenylpropanolamine, cetirizine, mixtures thereof and pharmaceutically acceptable salts thereof. It is preferred that the solid pharmaceutical (uncoated) have a particle size within the range of about 25 to about 250, preferably about 50 to about 175, microns.

The pharmaceutical is present in the freeze-dried dosage form in a therapeutic effective amount, which is an amount that produces the desired therapeutic response upon oral administration and can be readily determined by one skilled in the art. In determining such amounts, the particular compound being administered, the bioavailability characteristics of the pharmaceutical, the dose regime, the age and weight of the patient, and other factors must be considered.

The freeze-dried dosage form, on a dry basis, generally comprises from about 10 to about 90, preferably about 30 to about 60, percent by weight of the coated pharmaceutical particle and from about 10 to about 90, preferably about 30 to about 70, percent by weight of the water-soluble or water-dispersible carrier material.

The freeze-dried dosage form may also contain ingredients other than the carrier material and the coated particle. For example, the dosage form may incorporate pharmaceutically acceptable excipients. Such excipients, include, for example, preservatives, flavors, sweeteners and/or colorings.

Other pharmaceutically acceptable excipients which may be added to the current invention include suspending agents, such as natural and synthetic gums, maltodextrins, modified starch, cellulosic polymers, and synthetic polymers (Carbomer 934P); cationic, anionic and non-ionic surfactants (poloxamer(s), Tween(s), Span(s), Miglyols, Cremaphors); chealating salts and buffering agents/salts, such as edetate calcium disodium, edetate tetrasodium, and potassium phosphate(s); and/or antioxidants, such as Vitamin A, Vitamin E and butylated hydroxyanisole and butylated hydroxytoluene. One or more of the aforementioned excipients may be present in the dosage form, on a dry basis, in an amount ranging from about 0.05 to about 20, preferably about 0.1 to about 10, percent by weight of the dosage form.

The freeze-dried dosage forms of the present invention containing the coated pharmaceutical particles are prepared using freeze-drying or lyophilization processes generally known in the art. Such processes are described in U.S. Patent Nos. 4,305,502 and 4,371,516, both to Gregory, et al., and U.S. Patent No. 4,642,903 to Davies, all of which are incorporated by reference.

The freeze-dried dosage form is prepared by first coating pharmaceutical with the blend of the first and second polymer as previously described. A suspension of the coated pharmaceutical particles is then prepared by admixing the particles with a solution containing the water-soluble or water-dispersible carrier material, excipient(s), and a solvent that will dissolve and/or disperse the carrier material. Suitable solvents include water, alcohol and oils, such as cottonseed oil. Generally, this suspension contains from about 1 to about 30 weight percent of the coated pharmaceutical particles, from about 3 to about 10 weight percent of the carrier material, about 0.001 to about 10 weight percent adju-vant(s) and about 55 to about 95 weight percent solvent.

The suspension of the coated pharmaceutical particles is then transferred to a mold containing one or more depressions. The depression size may correspond to the desired size and shape of the final product or it can be significantly larger in which case the product is cut into the desired shape after the freeze-drying process. The molds may also be coated or lined for easy release of the freeze-dried dosage form. Preferred molds are made from talc filled polypropylene with a layer of silicone/si-methicone baked onto the surfaces of the mold in contact with the suspension.

The molds and the contents are then frozen in a cold gas freezing tunnel at a temperature of about -10 to about -90°C. The frozen units, either in the molds or loose in suitable retaining trays, are then subjected to a vacuum of about $2.5 \times 10^{-2}$ to about $7.5 \times 10^{-1}$ Torr to cause the solvent to sublime.

Once the freeze-drying process is complete, the freeze-dried dosage form is transferred to moisture-impervious packaging, such as blister packs. Alternatively, the suspension may be freeze-dried within the blister packs.

Specific embodiments of the present invention are illustrated by way of the following examples. This invention is not confined to the specific limitations set forth in these examples, but rather to the scope of the appended claims. Unless otherwise stated, the percentages and ratios given below are by weight.

## EXAMPLE 1

This Example discloses an inventive freeze-dried dosage form containing acetaminophen as the active ingredient.

A coating solution containing 10% by weight of a blend of cellulose acetate (CA 398-10) and polyvinyl pyrrolidone (Povidone K-29/32) was prepared with an acetone/methanol (80:20) solvent. The ratio of cellulose acetate to polyvinyl pyrrolidone was 85:15.

Four kilograms of acetaminophen (nominal particle size of 125 microns) was charged into a Wurster (bottom spray) fluidized bed coating apparatus. The acetaminophen was then placed in a fluidized state by a flow (100-130 ft.$^3$/min.) of air at a temperature of 40°C. The coating solution was then sprayed (atomization air pressure = 2 bar) onto the fluidized acetaminophen particles at a rate of 15 grams/min. until a coated particle containing approximately 20% by weight of the coating was obtained.

A suspension of the coated acetaminophen particles was then prepared to produce an 80 mg acetaminophen dosage form. The suspension had the following composition:

| Ingredients | % W/W |
|---|---|
| Coated Acetaminophen Particles (80% W/W potency) | 12.5 |
| Mannitol, USP | 2.5 |
| Gelatin, NF | 2.2 |
| Glycine, USP | 2.5 |
| Aspartame, NF | 0.75 |
| Simethicone, USP | 0.007 |
| Sodium Hydroxide, NF | 0.016 |
| Carbomer 934P, NF | 0.05 |
| Xanthan Gum, NF | 0.02 |
| Edetate Calcium Disodium, USP | 0.1 |
| Purified Water, USP | q.s. |
| | 100 |

Aliquots (0.8 Gm) of this suspension were dispensed into 1 ml capacity molds and frozen in a Cryo-Quick Freeze Tunnel (temperature range -65°C to -80°C, 15 minutes transit time). The frozen product (in molds) was placed on the pre-chilled shelves (-15°C) of a VirTis Genesis 25LL Freeze-dryer. Once a vacuum level of 5.0 x 10$^{-1}$ Torr was developed, the shelf temperature was raised to +20°C at a rate of 0.5°C/min. The system was maintained under vacuum at this shelf temperature for 2 hours. The freeze-drying process was terminated when a Pressure-Rise Test (1.0 x 10$^{-1}$ Torr pressure rise in a closed chamber over 30 seconds) was passed.

The finished freeze-dried dosage form possessed the sweet taste of aspartame and dispersed in the mouth in less than 10 seconds. There was no after-taste associated with the acetaminophen.

## COMPARATIVE EXAMPLE A

This Comparative Example discloses a freeze-dried dosage form containing spray-dried coated acetaminophen.

Micronized acetaminophen was passed through a 35 mesh (Tyler) screen. The screened acetaminophen (105 grams) was then dispersed in 600 grams of water using a homogenizer mixer. The dispersion was then mixed in a marine-type impeller mixer while adding 124.5 grams of ethyl cellulose NF (AQUACOAT brand marketed by FMC Corp.) and 7.5 grams of dibutyl sebecate.

The dispersion was then transferred to a Buchi Brinkman Model 190 Mini Spray Drier and spray dried. The resulting coated particle contained approximately 70 percent by weight of the coating. The operating conditions were as follows:

     Inlet Temp. - 212°C

     Lower Chamber Temp. - 105°C (during spraying)

     Spray Rate - 7.5 pump setting

     Air Flow - 700 fan setting

A suspension of the spray dried ethyl cellulose coated acetaminophen particles having the composition shown below was freeze-dried as in Example 1 to produce an 80 mg acetaminophen dosage form. All process variables (i.e., mixing time of the suspension, freezing conditions, freeze-drying conditions) were as in Example 1.

| Ingredients | % W/W |
|---|---|
| Spray-Dried Acetaminophen Particles (70% Potency) | 14.28 |
| Mannitol, USP | 2.5 |
| Gelatin, NF | 2.2 |
| Glycine, USP | 1.5 |
| Aspartame, NF | 0.75 |
| Simethicone, USP | 0.0050 |
| Sodium Hydroxide, NF | 0.013 |
| Carbomer 934P, NF | 0.040 |
| Xanthan Gum, NF | 0.010 |
| Edetate Calcium Disodium, USP | 0.15 |
| Purified Water, USP | q.s. |
| | 100 |

The finished freeze-dried dosage form possessed a bitter, acetaminophen taste, had a gummy mouthfeel, and did not disperse in the mouth within 10 seconds.

**EXAMPLE 2**

This Example discloses an inventive freeze-dried dosage form containing acetaminophen as the active ingredient. This dosage form was used in the taste test described below.

A suspension of the coated acetaminophen particles prepared in Example 1 was used to produce an 80 mg acetaminophen dosage form. The suspension had the following composition:

| Ingredients | % W/W |
|---|---|
| Coated Acetaminophen Particles (80% Potency) | 12.52 |
| Mannitol, USP | 2.5 |
| Gelatin, NF | 2.2 |
| Glycine, USP | 2.5 |
| Aspartame, NF | 0.75 |
| Simethicone, USP | 0.0070 |
| Sodium Hydroxide, NF | 0.016 |
| Carbomer 934P, NF | 0.050 |
| Xanthan Gum, NF | 0.020 |
| Edetate Calcium Disodium, USP | 0.10 |
| Artificial Wild Cherry Flavor | 1.8 |
| Natural and Artificial Alpine Creme Flavors | 0.72 |
| Artificial Strawberry Flavor | 0.72 |
| Coloring | 0.002 |
| Purified Water | q.s. |
| | 100 |

The suspension was processed as in Example 1 to produce a finished freeze-dried 80 mg acetaminophen dosage form.

**COMPARATIVE EXAMPLE B**

This Comparative Example discloses a freeze-dried dosage form containing spray-dried coated acetaminophen, which was used in the taste test described below.

A suspension of the spray-dried coated acetaminophen particles prepared in Comparative Example A was used to produce an 80 mg acetaminophen dosage form. The suspension had the following composition:

| Ingredients | % W/W |
|---|---|
| Spray-Dried Acetaminophen Particles (70% Potency) | 14.28 |
| Mannitol, USP | 2.5 |
| Gelatin, NF | 2.2 |
| Glycine, USP | 1.5 |
| Aspartame, NF | 0.75 |
| Simethicone, USP | 0.0050 |
| Sodium Hydroxide, NF | 0.013 |
| Carbomer 934P, NF | 0.040 |
| Xanthan Gum, NF | 0.010 |
| Edetate Calcium Disodium, USP | 0.15 |
| Natural Orange Flavor | 1.00 |
| Natural and Artificial Alpine Creme Flavors | 0.50 |
| Colorings | 0.0035 |
| Purified Water, USP | q.s. |
| | 100 |

The suspension formula was then processed as in Example 1 to produce a finished freeze-dried 80 mg acetaminophen dosage form.

**TASTE TEST**

A taste test was conducted on the dosage forms produced above in Example 2 and Comparative Example B. Trained human subjects from a sensory quality control team were used in the taste test. Initially, the subjects were given bitter caffeine solutions at various concentrations to determine their ability to detect bitterness levels. The subjects were then given two levels of acetaminophen in TYLENOL® Elixir to determine their ability to detect the bitterness of acetaminophen. Based upon these two preliminary evaluations, six subjects were selected for participation in the taste test. Each subject was asked to dissolve the freeze-dried dosage forms of Example 2 and Comparative Example B on their tongue, indicate which one was more bitter, and score relative levels of bitterness.

Five of the six subjects indicated that Example 2 was the less bitter freeze-dried dosage form. The average bitterness level for Comparative Example B was 2.25 times higher than the bitterness level for Example 2. The results of this taste test indicate that the freeze-dried dosage form of the present invention has superior taste-masking characteristics.

**EXAMPLE 3**

This Example discloses an inventive freeze-dried dosage form containing loperamide as the active ingredient.

Rotogranulated loperamide particles are prepared by charging 0.4 kilograms of loperamide powder (particle size less than 20 microns), 3.0 kilograms of confectioners' sugar, 0.2 kilogram of polyvinyl pyrrolidone (Povidone K29/32) and 0.1 kilogram of microcrystalline cellulose into a fluidized bed rotogranulator. These materials are rotogranulated with water to prepare granules in the size range of 100 to 400 microns. The granules are then dried in the rotogranulator at a temperature of about 50°C.

A coating solution containing 10% of a blend of cellulose acetate (CA 398-10) and polyvinyl pyrrolidone (Povidone K29/32) in an acetone/methanol (80:20) solvent is prepared. The ratio of cellulose acetate to polyvinyl pyrrolidone is 85:15.

The rotogranulated particles are then coated in a rotogranulator. The coating solution is sprayed onto the particles until the particle contains 15% by weight of the coating. The coated loperamide particles are then dried until substantially all the solvents were removed.

The coated rotogranulated particles (85% W/W potency) are then incorporated at 0.29% W/W into the vehicle system described in Example 1. The suspension formula is processed as in Example 1 to produce a finished freeze-dried 2 mg loperamide dosage form.

## EXAMPLE 4

This Example discloses an inventive freeze-dried dosage form containing famotidine as the active ingredient.

Rotogranulated famotidine is prepared by charging 0.4 kilogram of fine famotidine powder, 3.5 kilograms of lactose fine powder (impalpable) and 0.2 kilogram of hydroxypropyl methylcellulose into a rotogranulator. This mixture is rotogranulated with water to produce granules in the range of 100 to 350 microns. These particles are then dried in the rotogranulator at about 50°C.

A coating solution containing 10% of a blend of cellulose acetate (CA 398-10) and hydroxypropyl cellulose (KLUCEL EF) is prepared in an acetone/methanol (80:20) solvent.

The rotogranulated particles are then charged into a Wurster coater and a coating solution is sprayed until a loading level of 15% by weight is achieved.

The coated famotidine particles (85% W/W potency) are then incorporated at 2.94% W/W into the vehicle system described in Example 1. The suspension formula is processed as in Example 1 to produce a finished freeze-dried 20 mg famotidine dosage form.

## EXAMPLE 5

This Example discloses an inventive freeze-dried dosage form containing aspirin.

Four kilograms of granular aspirin (150 micron particle size) is charged into a Wurster fluidized bed apparatus. The coating solution containing cellulose acetate and polyvinyl pyrrolidone prepared in Example 1 is sprayed onto the aspirin until a coating level of 20% by weight is achieved.

The coated aspirin particles (80% W/W potency) are then incorporated at 15.6% W/W into the vehicle system described in Example 1. The suspension formula is processed as in Example 1 to produce a finished freeze-dried 100 mg aspirin dosage form.

Various modifications can be made from the above-described embodiments without departing from the spirit and scope of the present invention.

## Claims

1. A freeze-dried pharmaceutical dosage form comprising a porous matrix of a water-soluble or water-dispersible carrier material containing at least one coated particle, said coated particle comprising at least one pharmaceutical coated with a blend of a first polymer which is a cellulose acetate or cellulose acetate butyrate and a second polymer which is polyvinyl pyrrolidone or hydroxypropyl cellulose, wherein the weight ratio of the first polymer to the second polymer is within the range of about 90:10 to about 50:50.

2. The pharmaceutical dosage form of claim 1 wherein the coated particle comprises about 5 to 60 percent by weight of the blend of first and second polymers.

3. The pharmaceutical dosage form of claim 1 or claim 2 wherein the pharmaceutical is acetaminophen, ibuprofen, flurbiprofen, naproxen, aspirin, pseudoephedrine, chlorpheniraminemaleate, dextromethorphan, diphenhydramine, famotidine, loperamide, ranitidine, cimetidine, astemizole, terfenadine, terfenadine carboxylate, domperidone, phenylpropanolamine, cetirizine, a pharmaceutically acceptable salt thereof or a mixture thereof and wherein preferably the pharmaceutical is acetaminophen, loperamide, famotidine or aspirin.

4. The pharmaceutical dosage form of any one of claims 1 to 3 wherein the carrier material comprises gelatin, pectin, mannitol or a mixture thereof, and at least one amino acid having from about 2 to about 12 carbon atoms.

5. The pharmaceutical dosage form of claim 4 wherein said at least one amino acid is glycine.

6. The pharmaceutical dosage form of any one of claims 1 to 5 wherein the coated particle comprises acetaminophen coated with a blend of cellulose acetate and polyvinyl pyrrolidone, wherein the weight ratio of cellulose acetate to polyvinyl pyrrolidone is about 85:15.

7. The pharmaceutical dosage form of claim 6 wherein the coated particle comprises about 20 percent by weight of said blend.

8. The pharmaceutical dosage form of any one of claims 1 to 7 comprising from about 10 to about 90 percent of said coated particle, from about 10 to about 90 percent of said carrier material and from about 0.05 to about 20 percent of a pharmaceutically acceptable excipient, by weight of the total dosage form.

9. A process for preparing a freeze-dried pharmaceutical dosage form, comprising the steps of:
forming at least one coated particle by coating in a fluidized bed a pharmaceutical with a blend of a first polymer which is a cellulose acetate or cellulose acetate butyrate and a second polymer which is polyvinyl pyrrolidone or hydroxypropyl cellulose, wherein the weight ratio of the first polymer to the second polymer is within the range of about 90:10 to about 50:50;
forming a suspension of the coated pharmaceutical particle in a solution comprising a water-soluble or water-dispersible carrier material and a solvent; and
freeze-drying a unit volume of said suspension so as to form a porous matrix of the water-soluble or water-dispersible carrier material with said coated particle dispersed therethrough.

10. The process of claim 9, which produces a freeze-dried pharmaceutical dosage form according to any one of claims 1 to 8.

EP 0 636 365 A1

| | European Patent Office | EUROPEAN SEARCH REPORT | Application Number EP 94 30 5535 |

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | EP-A-0 317 274 (MC NEIL CONSUMER PRODUCTS COMPANY) 24 May 1989<br>* page 6; example 1 *<br>* page 8; example 7 *<br>* page 11; example 15 *<br>--- | 1-10 | A61K9/20<br>A61K9/50 |
| Y | EP-A-0 352 190 (FARMALYOC) 24 January 1990<br>* column 3, line 59 - column 4, line 31 *<br>* column 6, line 26 - line 33 *<br>* column 11 - column 12; example 9 *<br>* claim 1 *<br>--- | 1-3,6-10 | |
| Y | WO-A-91 09591 (MEDIVENTURES INC) 11 July 1991<br>* page 13, line 11 - line 16 *<br>* page 19; example 4 *<br>* page 51; example 36 *<br>--- | 1,4,5 | |
| A | WO-A-93 12770 (PFIZER INC) 8 July 1993<br>* page 9 - page 10; example 9 *<br>--- | 1-10 | TECHNICAL FIELDS SEARCHED (Int.Cl.6) |
| A | EP-A-0 459 695 (MC NEIL PPC INC) 4 December 1991<br>* page 5; example 1 *<br>----- | 1-10 | A61K |

The present search report has been drawn up for all claims

| Place of search | Date of completion of the search | Examiner |
|---|---|---|
| THE HAGUE | 18 November 1994 | Boulois, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P04C01)

12